Europäisches Patentamt

European Patent Office

Office européen des brevets

(11) Veröffentlichungsnummer: **0 212 159 B1**

## EUROPÄISCHE PATENTSCHRIFT

(45) Veröffentlichungstag der Patentschrift: **02.01.92**

(51) Int. Cl.⁵: **A61M 25/00**

(21) Anmeldenummer: **86108945.6**

(22) Anmeldetag: **01.07.86**

(54) **Gefässkatheter.**

(30) Priorität: **01.07.85 DE 3523520**

(43) Veröffentlichungstag der Anmeldung:
**04.03.87 Patentblatt 87/10**

(45) Bekanntmachung des Hinweises auf die Patenterteilung:
**02.01.92 Patentblatt 92/01**

(84) Benannte Vertragsstaaten:
**CH DE FR GB IT LI NL SE**

(56) Entgegenhaltungen:
DE-A- 2 007 738    GB-A- 1 511 547
US-A- 1 906 678    US-A- 3 314 430
US-A- 4 129 129    US-A- 4 186 745

PROSPEKT DER UNITED STATES CATHETER AND INSTRUMENT CORPORATION (USCI) "Cardiovascular Catheters and Accessories", 1969; New York

DIS. CHEST, Band 35, Mai 1959 G. BLANCO et al. "Single Catheter Gravity Drainage of the Right Atrium or Right Ventricle during Total Cardiac Bypass" Seiten 554-560

(73) Patentinhaber: **Stöckert Instrumente GmbH**
**Osterwaldstrasse 10**
**W-8000 München 40(DE)**

(72) Erfinder: **Kreuzer, Rudolf**
**Kreilerstrasse 7**
**W-8000 München 80(DE)**
Erfinder: **Stöckert, Friedemann**
**Emmeram 53**
**W-8000 München 81(DE)**
Erfinder: **Zupkas, Paul Francis**
**1590 Santa Ana Avenue**
**Costa Mesa California 92627(US)**
Erfinder: **Noda, Wayne Arthur**
**28072 Paseo Rincon**
**Mission Viejo California 92692(US)**

(74) Vertreter: **Eitle, Werner, Dipl.-Ing. et al**
**Hoffmann, Eitle & Partner Patentanwälte Arabellastrasse 4**
**W-8000 München 81(DE)**

EP 0 212 159 B1

**Beschreibung**

Die Erfindung betrifft einen Gefäßkatheter zur Kannulierung arterieller oder venöser Gefäße, z.B. im Femoralbereich oder der Vena Cava und bzw. oder des rechten Herzvorhofes, bestehend aus einem Schlauch mit lochartigen Blutdurchtritts-Öffnungen an seinem in das Gefäß bzw. den Herzvorhof einführbaren vorderen Schlauchende (Einführungsende), die durch einen an diesem Schlauchende befindlichen Schleißkörper verschließbar sind, welcher Schlauch mit seinem anderen Ende mittelbar oder unmittelbar an eine andere Leitung bzw. ein Gerät anschließbar ist.

Derartige Katheter werden u.a. als venöse Rückflußkatheter im Zusammenhang mit Herz-Lungen-Maschinen angewendet, welche bei Herzoperationen zeitweilig die Herz- und Lungenfunktion des Patienten übernehmen. Dabei dient der Katheter dazu, das zum Herzen des Patienten zurückfließende Blut aus dem rechten Herzvorhof bzw. der dort einmündenden inferior Vena Cava abzuziehen und der Herz-Lungen-Maschine bzw. der Pumpe für das Umpumpen des Blutes zuzuführen.

Durch die US-A-4 129 129 ist ein Rückflußkatheter der eingangs genannten Gattung bekanntgeworden, der an seiner Einführungsspitze einen Korb mit Bluteinlaßöffnungen und im Abstand von dieser Einführungsspitze einen zweiten Korb mit weiteren Bluteinlaßöffnungen besitzt. Mit seinem ersten Korb wird dieser Katheter durch den Herzvorhof hindurch in die Inferior Vena Cava eingeschoden, wobei der zweite Korb in dem rechten Herzvorhof zu liegen kommt. Dadurch wird einer durch Distorsion der Arterienwände und der Vena Cava oder durch Änderung der Katheterlage bedingten Reduktion der venösen Drainage begegnet.

Eine einzelne Katheterkannulierung ist auch in dem Aufsatz "Single Catheter Gravity Drainage of the Right Atrium or Right Ventricle During Total Cardiac Bypass" von G.C. Blanco u.a. in Dis. Chest, 35, Mai 1959, S. 554-560, beschrieben. Der auf Seite 555 dieser Literaturstelle gezeigte Katheter hat zahlreiche Perforationen im distalen Teil seines Körpers und wird so positioniert, daß diese Perforationen sich von dem rechten Atrium zu der Inferior vena cava erstrecken. Mit diesen Perforationen soll durch einen einzigen Katheter Blut sowohl von dem rechten Atrium als auch der Inferior vena cava aufgefangen werden.

Bei diesen und den meisten anderen bekannten Kathetern mit in Katheterlänge verteilt angeordneten Blutdurchtrittsöffnungen besteht jedoch das Problem, daß bereits bei Beginn des Einführens des Katheters in das Gefäß bzw. den Herzvorhof durch seine an der Einführungsspitze vordersten Öffnungen Blut in ihn eindringt, welches aus den dahinter folgenden Öffnungen sogleich in den Körper des Patienten austritt, bis der Katheter auch mit diesen Öffnungen in den Herzvorhof bzw. das Gefäß eingeschoben ist. Da das Einschieben des Katheters vorsichtig und langsam erfolgen muß, kann dabei eine erhebliche Blutmenge aus dem Gefäß bzw. dem Herzvorhof in den Körper des Patienten ausfließen.

Der Erfindung liegt daher die Aufgabe zugrunde, diesen Nachteil mit möglichst einfachen Mitteln zuverlässig auszuschalten. Dies wird erfindungsgemäß mit dem im kennzeichnenden Teil des Anspruchs 1 genannten Mitteln erreicht. Im Prinzip ist dabei, in Anpassung an die für Gefäßkatheter bestehenden Gegebenheiten, im Katheterschlauch ein kolbenartiger Schließkörper in Schlauchlängsrichtung derart hin- und herbewegbar, daß er in eine Lage im Schlauch zumindest einen Teil der Blutdurchtrittsöffnungen abdeckt und damit verschließt und in anderen Lagen diese Öffnungen freigibt. Zweckmäßig ist der Schließkörper so ausgebildet, daß er in seiner Schließlage sämtliche Blutdurchtrittsöffnungen im Einführungsende des Katheterschlauches abdeckt.

Die Anordnung von Schließkörpern in Kathetern als solche ist, z.B. aus der US-A-4.186.745, für Drainageröhren bekannt. Dort wird ein Ballon zum Schließen der Durchtrittsöffnungen aufgeblasen und zu deren Freigabe wieder entleert.

Mit der erfindungsgemäßen Katheterausbildung wird es jedoch möglich, ein Eindringen von Blut in das vordere Einführungsende mit der Gefahr des Wiederaustretens durch dahinter liegende Öffnungen während des Einführens des Katheters solange zu verhindern, bis der Katheter mit sämtlichen Blutdurchtrittsöffnungen in das Gefäß bzw. den Herzvorhof eingeschoben ist. Dies kann durch Schließen der Blutdurchtrittsöffnungen, zumindest der vordersten Blutdurchtrittsöffnungen, im Einführungsende des Katheterschlauches durch den darin verschiebbaren kolbenartigen Schließkörper geschehen. Nach dem vollständigen Einschieben des Katheters mit seinem Einführungsende in das Gefäß bzw. den Herzvorhof können die Blutdurchtrittsöffnungen durch Zurückziehen des Schließkörpers bzw. dessen Herausziehen aus dem Katheterschlauch geöffnet werden, wobei vor dem Herausziehen des Schließkörpers aus dem Katheterschlauch dieser an einer Stelle außerhalb des Gefäßes bzw. Herzens abgeklemmt werden kann. Um einen dichten Abschluß der Blutdurchtrittsöffnungen im Katheterschlauch sicherzustellen, sollte der Schließkörper in seiner die Öffnungen schließenden Lage an den Rändern der Öffnungen an der Schlauchwandung anliegen. Hierzu ist es zweckmäßig, daß die Seitenwand des Schließkörpers zur die Blutdurchtrittsöffnungen enthaltenden Seitenwand des Schlauchendes im wesentlichen parallel ist.

An den kolbenartigen Schließkörper ist vorteilhaft ein Betätigungskabel befestigt, das sich im Katheterschlauch etwa bis zu seinem dem Einführungsende entgegengesetzten offenen Schlauchende erstreckt bzw. an diesem Schlauchende aus dem Katheterschlauch herauserstreckt. Mit diesem Betätigungskabel kann der Schließkörper in seine Schließlage und aus dieser herausbewegt sowie gegebenenfalls aus dem Katheterschlauch herausgezogen werden.

Vorteilhaft ist im Katheterschlauch im Abstand von dem die Blutdurchtrittsöffnungen aufweisenden Einführungsende ein Abdichtpfropfen angeordnet, der verschiebbar auf dem Betätigungskabel sitzen und mit dem Schließkörper aus dem Katheterschlauch herausgezogen werden kann. Hierdurch wird selbst dann, wenn der Schließkörper kein völliges Abdecken und Schließen der Blutdurchtrittsöffnungen bewerkstelligt, vermieden und auch beim Herausbewegen des Schließkörpers aus seiner Schließlage im Katheterschlauch ausgeschlossen, daß Blut an seinem offenen hinteren Ende aus dem Katheterschlauch austritt, bevor dieser Schlauch an das Schlauchsystem der Herz-Lungen-Maschine oder ein anderes Gerät angeschlossen werden kann. Wird der Schließkörper aus dem Katheterschlauch über dieses offene hintere Schlauchende entfernt, ist ein Anschließen des Katheters an das Schlauchsystem od.dgl. erst nach der Entfernung des Schließkörpers möglich. Dabei kann der Schließkörper bei durch den vorgenannten Pfropfen abgedichtetem hinteren Schlauchende des Katheters bis zu diesem Pfropfen bewegt werden, worauf der Katherschlauch vor dem Schließkörper abgeklemmt werden kann, so daß dann Schließkörper mit Pfropfen gezogen werden können, ohne daß Blut aus dem Katheterschlauch vor dessen Anschluß an das Schlauchsystem od.dgl. herausläuft.

Derartiges ist bei dem Herzkatheter der US-A-1 129 129 nicht möglich, obwohl bei diesem Schlauch eine Einrichtung vorgesehen ist, mit welcher verhindert werden kann, daß durch den ersten Korb hindurch in die Katheterspitze eingedrungenes Blut während des Einführens des Katheters nicht durch den dahinter befindlichen zweiten Öffnungskorb wieder aus dem Katheterschlauch herausfließen kann. Diese bekannte Einrichtung besteht darin, daß das den ersten Öffnungskorb enthaltende vorderste Ende des Katheterschlauchs kleineren Durchmesser hat als der den zweiten Öffnungskorb enthaltende dahinter folgende Teil des Katheterschlauches und daß in diesen vordersten Katheterschlauch mit geringerem Durchmesser ein Abdichtschlauch eingeschoben werden kann, der das in die Katheterspitze eingetretene Blut von den Öffnungen des zweiten Korbes fernhält. Dieser Abdichtschlauch kann an seinem hinteren aus dem Katheter herausragenden Ende abgeklemmt werden und aus dem Katheterschlauch herausgezogen werden, wenn dieser auch mit seinem zweiten Öffnungskorb in den Herzvorhof bzw. die Vena Cava eingeschoben worden ist.

Da bei diesem bekannten Katheter der genannte Abdichtschlauch jedoch die Blutdurchtrittsöffnungen es zweiten Öffnungskorbes nicht abdichtet, gelangt durch diese Öffnungen hindurch Blut in den Katheter, sobald dieser mit diesem Öffnungskorb in den Herzvorhof bzw. die Vena Cava eingeschoben worden ist. Da vor dem Anschließen des Katheters an das Schlauchsystem der Herz-Lungen-Maschine oder ein anderes Gerät das hintere offene Katheterende nicht verschlossen sein darf, um den Abdichtschlauch vorher aus dem Katheter herausziehen zu können, ist es unvermeidlich, daß dieses in den Katheter eingedrungene Blut aus dem hinteren offenen Ende des Katheters herausläuft, bis dieses Katheterende an das Schlauchsystem od.dgl. angeschlossen worden ist.

Weitere vorteilhafte Ausgestaltungen des erfindungsgemäßen Katheters ergeben sich aus den Unteransprüchen.

In der Zeichnung sind besonders vorteilhafte Ausführungsbeispiele des erfindungsgemäßen Katheters dargestellt, welches im folgenden näher beschrieben wird.

Fig. 1 zeigt eine erste Ausführungsform in Seitenansicht teilweise geschnitten bei geöffneten Blutdurchtrittsöffnungen,

Fig. 2 ist ein Längsschnitt durch diese Ausführungsform bei verschlossenen Blutdurchtrittsöffnungen.

Fig.3 ist ein Längsschnitt durch diese Ausführungsform bei entferntem Schließkörper,

Fig. 4 zeigt den Schließkörper ohne den Katheterschlauch,

Fig. 5 zeigte eine Seitenansicht einer zweiten Ausführungsform, abgebrochen dargestellt und

Fig. 6 zeigt einen vergrößerten Querschnitt durch diese zweite Ausführungsform nach Linie 6-6 in Fig. 5.

Bei dem in der Zeichnung dargestellten Ausführungsbeispiel ist der Katheterschlauch 1 an seinem sich leicht verjüngenden Einführungsende 2 mit sich über seinen Umfang und über einen Teil seiner Katheterlänge gleichmäßig verteilten Blutdurchtrittsöffnungen 3 versehen. Der Katheterschlauch ist an seinem offenen hinteren Ende 4 durch einen Pfropfen 5 abgeschlossen, der verschiebbar an der Innenwand des Katheterschlauches dicht anliegt. Im Inneren des Katheterschlauches ist ein kolbenartiger Schließkörper 6 angeordnet, der in Längsrichtung des Katheterschlauches 1 in diesem hin- und herbewegbar ist. Zu diesem Zweck ist der Schließkörper an seinem rückwärtigen Ende mit einem Betätigungskabel 7 verbunden, das sich verschiebbar durch den Pfropfen 5 hindurch am hinteren offenen Ende 4 des Katheterschlauches 1 aus diesem heraus erstreckt und an seinem äußeren Ende mit einem Handgriff 8 versehen ist.

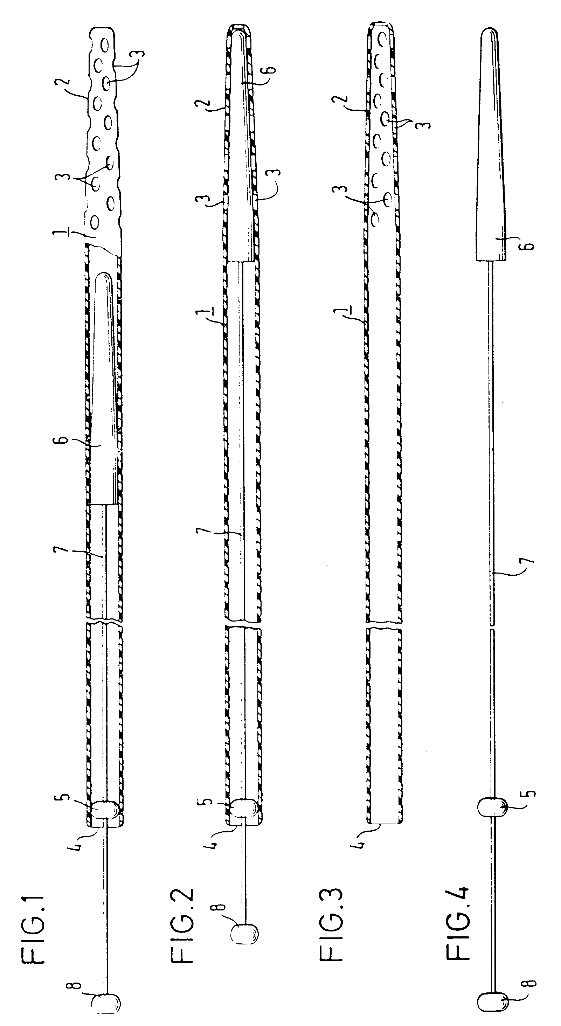

In Fig. 2 ist der Schließkörper 6 in seiner vordersten, die Blutdurchtrittsöffnungen 3 abdeckenden Lage gezeigt. Damit der Schließkörper in dieser Lage dicht an den Rändern aller Blutdurchtrittsöffnungen 3 anliegt, um diese Öffnungen verschließen zu können, ist er in seiner Form und seinen Abmessungen an diejenigen der Innenseite des Einführungsendes 2 des Katheterschlauches 1 angepaßt.Der kolbenartige Schließkörper hat eine ebenfalls konische Form, wobei sich dieser Schließkörper zu seinem dem Betätigungskabel 7 abgewandten vorderen Ende etwa parallel zur Konizität des Einführungsendes 3 des Katheterschlauches verjüngt. Die axiale Länge des Schließkörpers ist so bemessen, daß er sich in seiner in Fig. 2 gezeigten Schließlage über das gesamte die Durchtrittsöffnungen 3 enthaltende Einführungsende 2 des Katheterschlauches erstreckt. Die Blutdurchtrittsöffnungen 3 ihrerseits erstrecken sich über eine solche axiale Länge des Katheterschlauches 1 im Bereich seines Einführungsendes 2, die geringfügig kleiner als die in das Gefäß bzw. den Herzvorhof einschiebbare Katheterlänge ist.

Mit in Schließstellung gemäß Fig. 2 gebrachtem Schließkörper 6 wird der Katheter vorsichtig durch einen in der Gefäß- bzw. Herzwand gemachten Schnitt in das Gefäß bzw. den Herzvorhof soweit eingeschoben, bis sich sämtliche Blutdurchtrittsöffnungen innerhalb des Gefäßes bzw. Vorhofes befinden Durch die abgedichteten Blutdurchtrittsöffnungen kann kein Blut in den Katheterschlauch 1 gelangen. Sollte trotzdem eine geringe Blutmenge eindringen, wird diese im Katheterschlauch durch den Abdichtpfropfen 5 am Austreten gehindert.

Nach dem völligen Einschieben des Katheterschlauches mit seinem Einführungsende 2 kann der Schließkörper 6 mit Hilfe des Betätigungskabels 7 bis oder nahe bis zum Abdichtpfropfen 5 zurückgezogen werden, wodurch die Blutdurchtrittsöffnungen 3 freigelegt werden und durch diese hindurch Blut in den Katheterschlauch eintritt. Dieses Blut kann jedoch aufgrund des Abdichtpfropfens 5 und des zumindest teilweise an der Wandung des Katheterschlauches 1 anliegenden oder im wesentlichen anliegenden Schließkörpers nicht durch das offene Ende 4 des Katheterschlauches heraustreten. Nun kann der Katheterschlauch zwischen seinem in das Gefäß bzw. den Herzvorhof eingeschobenen Einführungsende und dem zurückgezogenen Schließkörper 6 abgeklemmt werden, worauf Schließkörper 6 mit Abdichtpfropfen 5 aus dem Katheterschlauch 1 herausgezogen und dieser mit seinem hinteren offenen Ende an ein Schlauchsystem bzw. an ein Gerät, z.B. eine Flüssigkeitspumpe, angeschlossen werden kann.

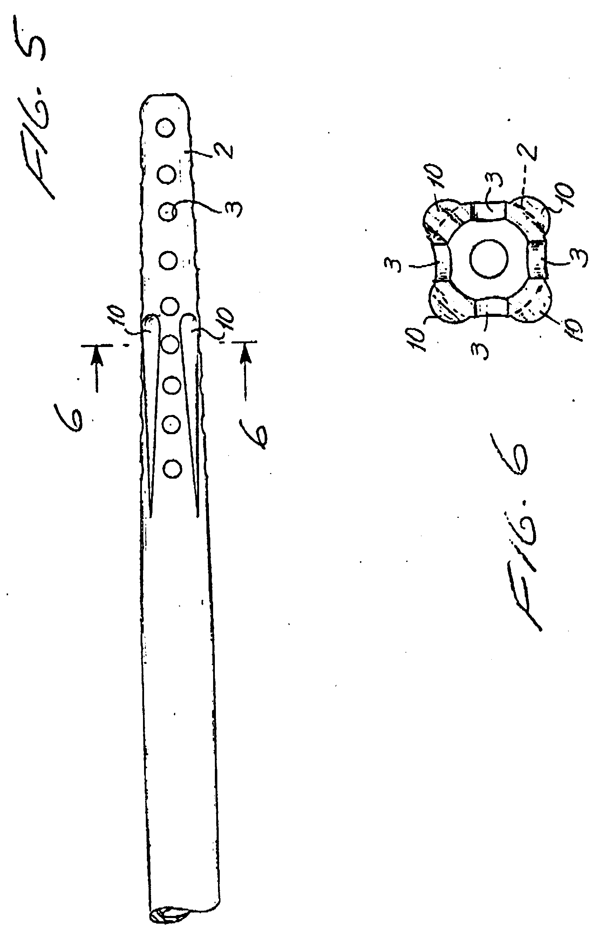

Fig. 5 und 6 veranschaulichen eine weitere Ausführungsform der Erfindung. Bei dieser Ausführungsform besitzt der Katheterschlauch eine Anzahl von in ihm ausgebildeten integralen länglichen Rippen 10, die in der Nähe des vorderen Schlauchendes über den Schlauchumfang gleichmäßig verteilt angeordnet sind. Diese Rippen 10 dienen der Verstärkung und Stabilisierung des Katheterschlauches und verhindern, daß das den Schlauch umgebende Gewebe die Blutdurchtrittsöffnungen 3 im Schlauch okkludiert.

**Patentansprüche**

1. Gefäßkatheter zur Kanulierung arterieller oder venöser Gefäße im Femoralbereich oder der Vena Cava und bzw. oder des rechten Herzvorhofes, bestehend aus einem Schlauch (1) mit lochartigen Blutdurchtritts-Öffnungen (3) an seinem in das Gefäß bzw. den Herzvorhof einzuführenden vorderen Schlauchende (2), die durch einen in diesem Schlauchende befindlichen Schließkörper (6) verschließbar sind, welcher Schlauch mit seinem hinteren offenen Ende mittelbar oder unmittelbar an eine andere Leitung oder ein Gerät anschließbar ist, dadurch gekennzeichnet, daß die Blutdurchtrittsöffnungen (3) in dem Schlauchende (2) an dessen Seitenwand über den Schlauchumfang und über einen Teil der Schlauchlänge verteilt angeordnet sind, welcher Teil das 3- bis 10-fache des Durchmessers bzw. des mittleren Durchmessers des die Öffnungen enthaltenden Schlauchendes beträgt, und daß der Schließkörper (6) in Richtung der Schlauchlänge eine Länge hat, die größer ist als die Länge des die Blutdurchtrittsöffnungen (3) enthaltenden Schlauchendes (2) ist, und in Schlauchlängsrichtung derart kolbenartig hin- und herbewegbar ist, daß er in einer Lage im Schlauch die Blutdurchtrittsöffnungen (3) abdeckt und damit verschließt und in einer anderen Lage diese Öffnungen freigibt.

2. Katheter nach Anspruch 1, dadurch gekennzeichnet, daß die Blutdurchtrittsöffnungen (3) in der Schlauchseitenwand sich über einen Teil der gesamten Länge des Katheterschlauches (1) erstrecken, der gerüngfügig kleiner als die in das Gefäß bzw. den Herzvorhof einschiebbare Katheterlänge ist.

3. Katheter nach Anspruch 1, dadurch gekennzeichnet, daß der Schließkörper (6) so geformt und bemessen ist, daß er in seiner die Blutdurchtrittsöffnungen (3) schließenden Lage an den Rändern dieser Öffnungen an der Schlauchwand anliegt.

4. Katheter nach Anspruch 1, dadurch gekennzeichnet, daß der Schlauch (1) eine Mehrzahl von länglichen Rippen (10) aufweist, die am vorderen Ende des Schlauches über dessen Umfang verteilt angeordnet sind.

5. Katheter nach Anspruch 1, dadurch gekennzeichnet, daß das die Blutdurchtrittsöffnungen (3) enthaltende Einführungsende (2) des Schlauches (1) sich bis zu dessen Stirnende leicht konisch verjüngt.

6. Katheter nach Anspruch 5, dadurch gekennzeichnet, daß der kolbenartige Schließkörper (6) sich parallel zur Konizität des Einführungsendes (2) des Schlauches (1) verjüngt.

7. Katheter nach Anspruch 6, dadurch gekennzeichnet, daß das dem Einführungsende (2) entgegengesetzte Schlauchende eine das Herausziehen des Schließkörpers zulassende Öffnung (4) aufweist.

8. Katheter nach Anspruch 1, dadurch gekennzeichnet, daß an den kolbenartigen Schließkörper (6) ein Betätigungskabel (7) befestigt ist, das sich im Katheterschlauch (1) etwa bis zu dessen seinem Einführungsende entgegengesetzten offenen Schlauchende (4) erstreckt bzw. an diesem Schlauchende aus dem Katheterschlauch herauserstreckt.

9. Katheter nach Anspruch 8, dadurch gekennzeichnet, daß im Katheterschlauch (1) im Abstand von dem die Blutdurchtrittsöffnungen (3) aufweisenden Einführungsende (2) ein Abdichtpfropfen (5) angeordnet ist, der dicht an der Schlauchwand anliegt.

10. Katheter nach Anspruch 9, dadurch gekennzeichnet, daß der Abdichtpfropfen (5) verschiebbar auf dem Betätigungskabel (7) sitzt.

11. Katheter nach Anspruch 9, dadurch gekennzeichnet, daß der Abdichtpfropfen (5) mit dem Schließkörper (6) aus dem Katheterschlauch (1) herausziehbar ist.

## Claims

1. Vascular catheter for cannulation of arterial or venous vessels in the femoral area or the vena cava and/or the right atrium, consisting of a tube (1) with hole-like blood passage openings (3) at its front tube end (2) to be inserted into the vessel or the atrium, which openings can be closed off by means of a closure element (6) situated in this tube end, which tube can be connected via its rear open end directly or indirectly to another line or a device, characterised in that the blood passage openings (3) in the tube end (2) are arranged on its side wall, distributed over the tube periphery and over a part of the tube length, which part amounts to 3 to 10 times the diameter or the mean diameter of the tube end containing the openings, and in that the closure element (6) has, in the direction of the tube length, a length which is greater than the length of the tube end (2) containing the blood passage openings (3), and can be moved to and fro in the manner of a piston in the longitudinal direction of the tube in such a way that, in one position in the tube, it covers and thus closes the blood passage openings (3) and, in another position, it frees these openings.

2. Catheter according to Claim 1, characterised in that the blood passage openings (3) in the side wall of the tube extend over a part of the whole length of the catheter tube (1), which part is slightly smaller than the catheter length which can be inserted into the vessel or the atrium.

3. Catheter according to Claim 1, characterised in that the closure element (6) is shaped and dimensioned in such a way that, in its position closing the blood passage openings (3), it bears against the tube wall at the edges of these openings.

4. Catheter according to Claim 1, characterised in that the tube (1) has a plurality of elongate ribs (10), which are arranged at the front end of the tube, distributed over its periphery.

5. Catheter according to Claim 1, characterised in that the insertion end (2) of the tube (1) containing the blood passage openings (3) tapers slightly conically towards its end face.

6. Catheter according to Claim 5, characterised in that the piston-like closure element (6) tapers in parallel with the conicity of the insertion end (2) of the tube (1).

7. Catheter according to Claim 6, characterised in that the tube end opposite the insertion end (2) has an opening (4) allowing the closure element to be pulled out.

8. Catheter according to Claim 1, characterised in that an actuating cable (7) is secured on the piston-like closure element (6), which cable extends in the catheter tube (1) approximately to its open tube end (4) opposite its insertion end, or extends out from the catheter tube at this tube end.

9. Catheter according to Claim 8, characterised in that a sealing plug (5) is arranged in the catheter tube (1) at a distance from the insertion end (2) having the blood passage openings (3), which sealing plug bears tightly against the tube wall.

10. Catheter according to Claim 9, characterised in that the sealing plug (5) sits displaceably on the actuating cable (7).

11. Catheter according to Claim 9, characterised in that the sealing plug (5) can be pulled out from the catheter tube (1) together with the closure element (6).

**Revendications**

1. Cathéter destiné à être introduit à la manière d'une canule dans des vaisseaux artériels ou veineux de la région fémorale ou dans la veine cave et/ou dans l'oreillette droite du coeur, se composant d'un tuyau souple (1) dont l'extrémité antérieure (2) destinée à être introduite dans le vaisseau ou l'oreillette comporte des ouvertures (3) en forme de trous de passage du sang qui peuvent être fermées par un obturateur (6) se trouvant dans cette extrémité du tuyau, l'extrémité ouverte arrière de ce tuyau pouvant se raccorder indirectement ou directement à un autre conduit ou à un appareil, caractérisé en ce que les trous (3) de passage du sang situés à l'extrémité (2) du tuyau sont répartis dans la paroi latérale de cette dernière sur la circonférence du tuyau et sur une partie de la longueur de ce dernier, la longueur de cette partie étant égale à 3 à 10 fois le diamètre ou la moyenne du diamètre de l'extrémité du tuyau qui comporte les trous, et en ce que l'obturateur (6) a, dans la direction de la longueur du tuyau, une longueur qui est supérieure à la longueur de l'extrémité (2) du tuyau qui comporte les trous (3) de passage du sang et il est mobile alternativement à la manière d'un piston dans la direction de la longueur du tuyau de manière que, lorsqu'il occupe une position dans le tuyau, il recouvre les trous (3) de passage du sang et donc les obture et, lorsqu'il est à une autre position, il libère ces trous.

2. Cathéter selon la revendication 1, caractérisé en ce que les trous (3) de passage du sang situés dans la paroi latérale du tuyau sont placés sur une partie de la longueur totale du tuyau (1) du cathéter qui est légèrement inférieure à la longueur du cathéter pouvant être introduite dans le vaisseau ou l'oreillette du coeur.

3. Cathéter selon la revendication 1, caractérisé en ce que l'obturateur (6) est conformé et dimensionné de manière que, lorsqu'il est à la position de fermeture des trous (3) de passage du sang, il est appliqué sur les bords de ces trous contre la paroi du tuyau.

4. Cathéter selon la revendication 1, caractérisé en ce que le tuyau (1) comprend de multiples nervures longitudinales (10) qui sont réparties sur la circonférence de l'extrémité antérieure du tuyau.

5. Cathéter selon la revendication 1, caractérisé en ce que l'extrémité (2) d'introduction du tuyau (1) qui comporte les trous (3) de passage du sang se rétrécit légèrement en cône jusqu'au bout de cette extrémité.

6. Cathéter selon la revendication 5, caractérisé en ce que l'obturateur (6) en forme de piston se rétrécit parallèlement à la conicité de l'extrémité (2) d'introduction du tuyau (1).

7. Cathéter selon la revendication 6, caractérisé en ce que l'extrémité du tuyau opposée à l'extrémité (2) d'introduction présente une ouverture (4) qui autorise l'extraction de l'obturateur.

8. Cathéter selon la revendication 1, caractérisé en ce qu'un câble de manoeuvre (7) fixé à l'obturateur (6) en forme de piston se prolonge dans le tuyau (1) du cathéter approximativement jusqu'à l'extrémité ouverte (4) de ce dernier qui est opposée à l'extrémité d'introduction ou se prolonge au-delà de cette extrémité et ressort du tuyau du cathéter.

9. Cathéter selon la revendication 8, caractérisé en ce qu'un bouchon d'étanchéité (5) appliqué étroitement contre la paroi du tuyau est disposé dans le tuyau (1) du cathéter à distance de l'extrémité (2) d'introduction qui comporte les trous (3) de passage du sang.

10. Cathéter selon la revendication 9, caractérisé en ce que le bouchon d'étanchéité (5) est monté déplaçable sur le câble de manoeuvre (7).

11. Cathéter selon la revendication 9, caractérisé en ce que le bouchon d'étanchéité (5) peut être extrait du tuyau (1) du cathéter avec l'obturateur (6).

FIG.1

FIG.2

FIG.3

FIG.4

FIG. 5

FIG. 6

EP 0 212 159 B1